# EUROPEAN PATENT SPECIFICATION

(11) **EP 1 744 799 B1**
(45) Date of publication and mention of the grant of the patent: **28.06.2017**
(21) Application number: 05738600.5
(22) Date of filing: 27.04.2005
(51) Int. Cl.: A61M 5/32

(54) **APPARATUS FOR REMOVING NEEDLE ASSEMBLY**
VORRICHTUNG ZUM ENTFERNEN DER NADEL
SYSTEME POUR RETIRER UN ENSEMBLE AIGUILLE

(30) Priority: 27.04.2004 GB 0409354
(43) Date of publication of application: 24.01.2007
(73) Proprietor: OWEN MUMFORD LIMITED, Woodstock, Oxfordshire OX20 1TU (GB)
(72) Inventor: ROLFE, Steven Mark Guy, Oxfordshire OX27 8DF (GB); HUDSON, Christopher William, Oxfordshire OX29 4PP (GB)
(74) Representative: Branderhorst, Matthijs Pieter Arie
(86) International application number: PCT/GB2005/050058
(87) International publication number: WO 2005/102424

(56) References cited:
- WO-A-91/08786
- FR-A- 2 649 893
- US-A- 4 917 243
- US-A- 5 046 612
- US-A- 5 242 421

## Description

The present invention relates to an apparatus, a system and a method for achieving the safe removal of needles and in particular, though not necessarily, of hypodermic needles from injecting devices.

A range of drug injecting devices are currently available which comprise an outer reuseable housing into which is loaded a single or multi-dose drug containing cartridge. A hypodermic needle assembly is then attached to the cartridge through an end of the housing. An example of such a device is the Autopen™ injecting device available from Owen Mumford Ltd. of Woodstock, UK (see http://www.owenmumford.com). This device allows the dose to be delivered to be set using a dial mechanism. The force required to eject the drug from the cartridge is provided by a spring mechanism.

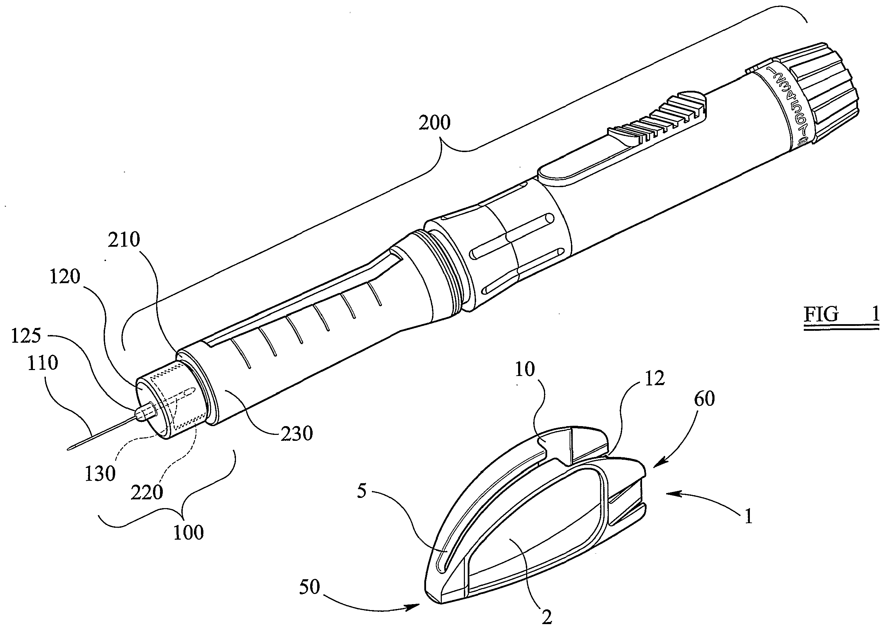

Considering in more detail the needle assemblies used with these injecting devices, a typical needle assembly is shown in Fig. 1 as 100 (top half of the figure). This needle assembly 100 comprises a stainless steel hypodermic needle 110 which is moulded into a generally cup-shaped plastics carrier 120, often referred to as a "hub". The needle 110 extends axially through the hub 120 so that a shorter portion 130 (shown in dashed lines in Fig. 1) is contained within the hub 120 whilst a longer portion projects outwardly. A screw thread is formed around the inner surface of the hub. This thread engages a corresponding external thread on an end of the injecting device containing the cartridge (both threads are indicated in dashed lines as 220 in Fig. 1). The action of coupling the screw threads causes the end of the needle 130 within the cup to penetrate a membrane provided on the cartridge, thereby coupling the drug contents to the passage extending through the needle. Fig. 1 also shows a typical injecting device 200 to which the disposable needle arrangement 100 has been mounted. Needle assemblies of the type shown in Fig. 1 are available from Owen Mumford Ltd. of Woodstock, UK (http://www.owenmumford.com).

Used needles should be disposed of safely. The disposal should be simple and easy to encourage safe disposal. The disposable needle assemblies 100 are normally sold with a protective cap (not shown) which is pushed over the small protrusion 125 forming part of the hub 120 and being located at the distal end of the hub 120 (in this specification the axial ends of the various devices shown in the drawings will be referred to as "distal end" and "proximal end" whereby the distal end is shown at the left in Figure 1 and the proximal end is shown at the right in Figure 1). Further, the entire needle assembly (with protective cap in place) is usually packaged in a sterile container which is pushed over hub 120 and extends axially in distal direction so as to protect the needle and cap. The proximal end of the container is foil-sealed.

In use, a user removes the foil seal and threads the hub onto the injecting device. The user then removes the container and protective cap and then performs an injection. After the injection, in many instances users push the container back onto the hub 120 by means of a relative axial movement and then rotate the injecting device 200 with respect to hub 120 so as to loosen the threaded connection. The needle assembly 100 within the container can then be disposed of. However, the relative axial movement between the needle assembly 100 and the protective cap often results in injury if needle 110 and the container are not properly aligned.

The present invention has been made in the light of this problem. The present inventors have devised an apparatus which enables the needle arrangement 100 to be re-capped without using the axial movement used in the past.

US 4,917,243 discloses a needle disposal device. US 5,242,421 discloses a needle cap. WO 91/08786 discloses a hypodermic needle sheath. US 5,046,612 discloses a safety apparatus for extracting hypodermic needles from the respective syringe. FR 2 649 893 discloses a disposable cap for a used hypodermic needle.

The present invention provides an apparatus and a method as defined in the accompanying claims. Further herein disclosed is an apparatus for removing a needle assembly from an injecting device, the needle assembly comprising a needle holder portion and a needle portion secured to, or integral with, the needle holder portion, the needle assembly being attachable to the injecting device via the needle holder portion, the apparatus comprising
a housing having an opening through which the needle assembly can enter the interior of the housing, wherein within the housing there is provided a receiving portion which is arranged to receive the needle holder portion in such a way as to impede rotation of the needle holder portion with respect to the receiving portion about the axis of the needle, and wherein the opening is formed such that the needle portion can enter the housing and the needle holder portion can enter the receiving portion by means of a relative movement between the housing and the needle portion which relative movement is lateral with respect to the axis of the needle portion, wherein the apparatus is formed such that said rotation can be impeded or further impeded by the application of lateral pressure onto the apparatus.

Further herein disclosed is a system comprising the above apparatus and a said needle assembly.

Further herein disclosed is a method of sheathing a needle assembly attached to an injecting device, comprising:
providing the above system;
performing a relative movement between the housing and the needle portion which relative movement is lateral with respect to the axis of the needle portion so that the needle assembly enters the interior of the housing, and so that the needle holder portion enters the receiving portion by means of said relative movement.

Method aspects corresponding to apparatus aspects disclosed herein are also provided, and vice versa.

Some preferred embodiments of the invention will now be described by way of example only and with reference to the accompanying drawings, in which:
Fig. 1 shows a perspective view of an injecting device to which a needle assembly has been mounted, and a needle removing apparatus.
Fig. 2 shows the injecting device and needle assembly of Fig. 1 inserted into the apparatus shown in Fig. 1.
Fig. 3 shows a top view of the items shown in Fig. 2.
Fig. 4 shows a perspective, more detailed view of a needle removing apparatus.
Fig. 5 shows a top view of the embodiment of Figure 4.
Fig. 6 shows a simplified cross-sectional view of a second needle removing apparatus.
Fig. 7 shows a perspective view of an embodiment of the apparatus of the present invention, in a first, open configuration.
Fig. 8 shows a side view of the embodiment of Figure 7.
Fig. 9 shows a top view of the embodiment of Figure 7.
Fig. 10 shows a perspective view of the embodiment of Figure 7, in a second, closed configuration.

Figure 1 shows a conventional injecting device 200 to which is mounted a needle assembly 100 comprising a cup-shaped hub 120 with a small protrusion 125 at the distal end of the hub 120 and carrying a needle 110. The hub 120 is formed with an internal thread 220 by means of which the hub 120 is threaded onto injecting device 200 which is formed with a corresponding external thread 220 at its distal end. "Behind" the threaded portion 220 of injection device 200 (i.e. adjacent to the threaded portion 220 in proximal direction) is a front portion 230 of the injecting device 200, which has a slightly larger diameter than the threaded portion 220. There is substantially no gap between the proximal end surface of the needle assembly 100 and the distal end surface 210 of front portion 230 of the injecting device 200, i.e. for practical purposes the proximal end surface of the needle assembly 100 lies flush against surface 210, so it is practically impossible for any tools to be applied between the proximal end surface of the needle assembly 100 and the distal end surface 210 of the enlarged front portion 230 of injecting device 200).

Figure 1 also shows a needle removing apparatus. Apparatus 1 is generally oblong to suit the dimensions of a needle assembly with which it is intended to be used. Apparatus 1 comprises a housing 2, which is preferably moulded in one piece from a slightly elastic plastics material, such as polypropylene, polyethylene, acetal or ABS. The housing 2 is essentially closed at its distal axial end 50 and open at its proximal axial end 60. It is further formed with a lateral slot 5, 10, 12 which has a relatively narrow slot portion 5 towards the distal end of apparatus 1, a wider slot portion 10 generally in the middle of apparatus 1 and an even wider slot portion 12 towards the proximal end of apparatus 1. The slot portions 5, 10 and 12 and the axial opening at the proximal end of the apparatus 1 form a continuous opening. The distal slot portion 5 is slightly longer than the exposed portion 110 of the needle, the middle slot portion 10 is approximately of the same length in axial direction as the hub 120, and the proximal slot portion 12 is approximately of equal length as slot portion 10, although various other lengths can be used.

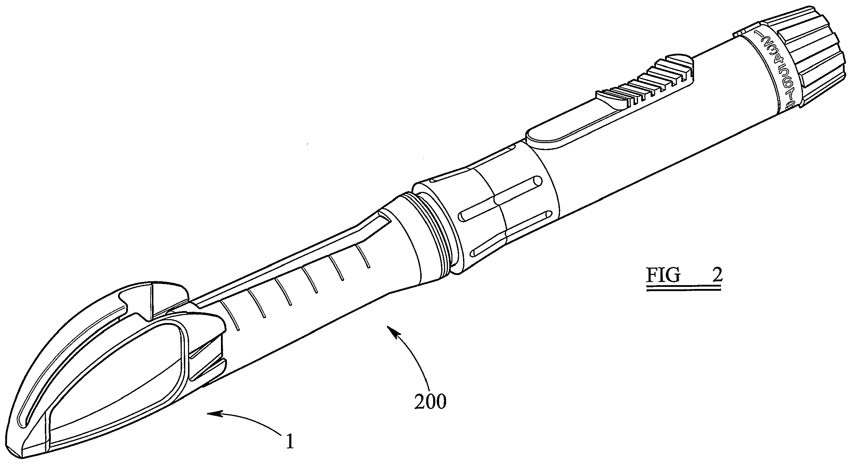

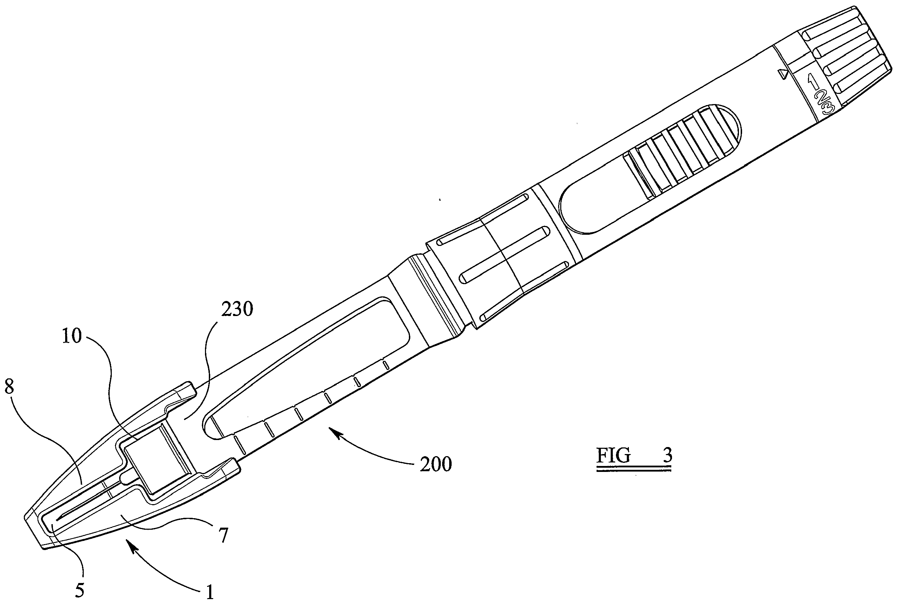

In the intended use, the injecting device 200 with mounted needle assembly 100 is approached to the apparatus 1 by means of a relative movement which is lateral with respect to the axis of needle 110. By means of this lateral movement, the needle assembly 100 and a portion of front portion 230 of injecting device 200 enters the housing 2 laterally until the injecting device 200 with mounted needle assembly 100 is secured to apparatus 1 as shown in Figure 2. The relative movement is preferably such that the needle 110 passes through distal slot portion 5, hub 120 passes through middle slot portion 10 and a portion of front portion 230 of injecting device 200 passes through proximal slot portion 12 of housing 2. Figure 3 shows a top view of the injecting device 200 with mounted needle assembly 100 secured to apparatus 1.

An industry standard for the external thread 220 of injecting device 200 has an overall diameter of about 9mm. The external diameter and any surface features of hub 120 vary from manufacturer to manufacturer. Needle assemblies sold by the Applicant have a hub with an external diameter of about 11.5mm with a plurality of longitudinally extending shallow grooves arranged around the circumference of the hub. One needle removing apparatus has a distal slot portion 5 which is about 3 to 4mm wide, a middle slot portion 10 which is approximately 11mm wide and a proximal slot portion 12 which is approximately 12 to 13mm wide. These dimensions may ensure that the needle assembly 100 can be inserted into the apparatus 1 with relative ease, whilst ensuring that it is virtually impossible for a user's fingers to enter the distal slot portion 5.

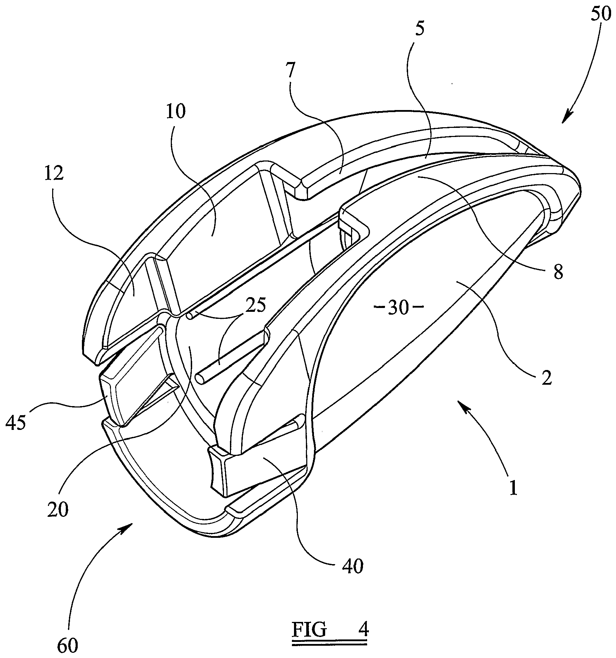

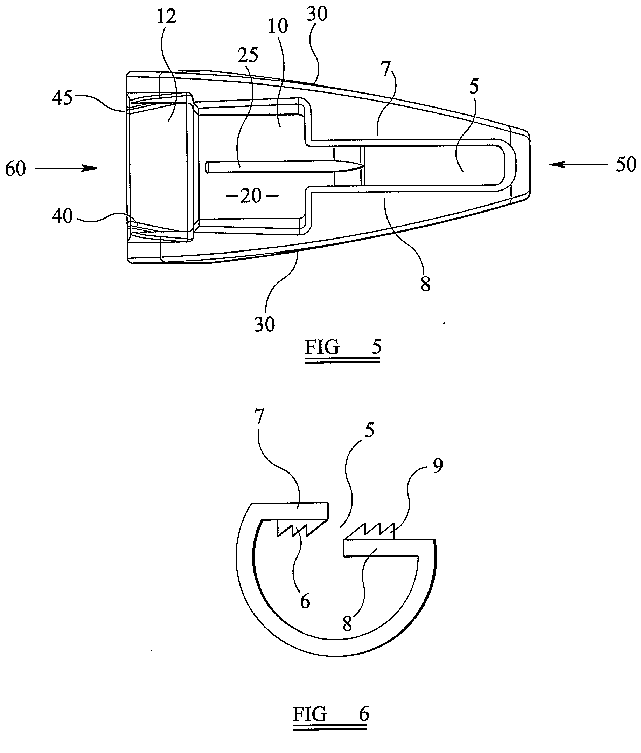

The thread of the injecting device has an external diameter of 9.5mm and has a pitch of 32 threads per inch (32 threads per 2.54cm). The thread may comply with ISO 11608. Referring now to Figures 4 and 5, the internal structure of apparatus 1 is shown in more detail. A generally cylindrical receiving portion 20 is formed in the housing at the same axial position as middle slot portion 10. This receiving portion 20 is intended to receive, surround and grip hub 120 so as to impede rotation of hub 120 with respect to the receiving portion. During insertion of needle assembly 100 into the apparatus 1 by means of the lateral movement the hub passes through middle slot portion 10, and moves further into receiving portion 20 by means of the lateral movement. The width of middle slot portion 10 is preferably slightly smaller than the diameter of receiving portion 20 so as to improve retention of hub 120 within housing 1. As the dimensions of the outer diameter of hub 120 vary from one manufacturer to the other, the housing 2 is preferably slightly elastic. This enables the middle slot portion 10 to be slightly dilated during lateral entry of hub 120.

The elasticity of housing 2 also enables sides 30 to be squeezed together slightly. This may further improve the grip of hub 120 within receiving portion 20. This can be of particular significance if the hub 120 has a particularly small external diameter. The elasticity of housing 2 also enables the apparatus to be used in conjunction with injecting devices 200 which have a relatively large external diameter of front portion 230 as proximal slot portion 12 can also be dilated elastically to a certain extent.

Receiving portion 20 has further at least one, but preferably several surface features which improve the grip of hub 120 within receiving portion 20. One such possible surface feature is shown in Figure 4 as longitudinal ribs 25. These may engage with any surface features on hub 120, such as grooves, or ribs. A variety of other surface features 25 may be provided, including grooves or a matrix of dots.

It is to be noted that, upon proper insertion of needle assembly 100 the needle assembly 100 is entirely surrounded by housing 2 (although of course the opening 5, 10, 12, remains open, but no portion of needle assembly 100 would project out of a fully convex surface wrapped around apparatus 1 after insertion of needle assembly 100 into housing 2).

In proper use, once the hub 120 is located in receiving portion 20, the housing 2 can be held by one hand of the user and the injecting device 200 by the other hand of the user, and the apparatus 1 rotated with respect to injecting device 200 so as to loosen the threaded connection between the hub and the injecting device. Since the receiving portion 20 prevents (or at least impedes) rotation (about the axis of the needle assembly 100) of hub 120 with respect to housing 2, the needle assembly 100 remains (relatively) stationary with respect to housing 2. Once the threaded connection between hub 120 and injecting device 200 is fully undone the injecting device 200 can be moved in proximal axial direction so as to retract front portion 230 of injecting device 200 out of proximal slot portion 12 of apparatus 1. The needle assembly 100 is thus separated from injecting device 200 and remains within apparatus 1. Assuming that the dimensions of hub 120 and receiving portion 20 are chosen suitably, the needle assembly 100 will be held securely within apparatus 1 and apparatus 1 can be disposed of, together with needle assembly 100 held within its housing 2.

Preferably, a relatively sharp edge is provided at the transition between middle slot portion 10 and receiving portion 20. This further improves the grip of hub 120 within receiving portion 20 and may help to prevent accidental or deliberate removal of needle assembly 100 from within housing 2 (it will be appreciated that removal of needle assembly 100 from housing 2 could present a health risk, both in terms of injury through sharp needle 110 and infection from a used needle).

A further feature of the needle removing apparatus shown in Figure 4 hinders removal of needle assembly 100 from apparatus 1: whilst at the proximal slot portion 12 the housing is formed generally cylindrical (the proximal slot portion 12 being provided in the lateral surface of the cylinder), two "legs" 40 and 45 are provided as resiliently deformable, radially inwardly projecting portions. The distal end of legs 40 and 45 is secured to receiving portion 20, while the proximal end of legs 40 and 45 projects inwardly. In the relaxed state of the apparatus 1 (i.e. without deformation caused by external forces or by insertion of injecting device 200) the distance between the proximal ends of legs 40 and 45 is smaller than the outer diameter of front portion 230 of a standard injecting device. On the other hand, the legs 40 and 45 are outwardly displaceable, (or pivotable) so that they are biased towards each other.

In normal use, insertion of injecting device 200 with needle assembly 100 into apparatus 1 by means of a lateral relative movement displaces legs 40 and 45 outwardly. The resulting bias of legs 40 and 45 does substantially not impede lateral entry of front portion 230 of injecting device 200 into proximal slot portion 12. Retraction of the injecting device 200 from apparatus 1 by unthreading the connection between hub 120 and injecting device 200 is also not substantially impeded. However, once the injecting device 200 is separated from hub 120 held within housing 2 legs 40 and 45 relax inwardly to their initial position, and since the distance between the proximal ends of legs 40 and 45 is slightly smaller than the outer diameter of front portion 230 of injecting device 200 any axial re-connecting of injecting device 200 to hub 120 is made substantially difficult.

It will be appreciated that only one, or more than two legs or other projections may be provided. In general terms, the diameter of the largest circle that can be placed between the proximal ends of the two or more legs 40, 45 should be smaller than the external diameter of the front portion 230 of injecting device 200.

In view of the different dimensions of hubs produced by various manufacturers, and further in view of the fact that the present apparatus may also find application with the removal of other needles, (such as the removal of needles from "traditional" syringes) it may be desirable to make apparatus 1 elastic to such an extent that it can be substantially deformed. The needle removing apparatus shown in Figure 4 may not allow a large degree of deformation since upon lateral pressure onto sides 30 the opposing lips 7 and 8 forming distal slot portion 5 will abut so as to close distal slot portion 5, after which no further deformation can be achieved by pressure onto sides 30. To this end it is possible, according to a second needle removing apparatus, to provide the lips 7 and 8 at different radial positions. As a result, sides 30 can be squeezed further, with lips 7 and 8 overlapping. This is illustrated in the simplified cross section shown in Figure 6. As a further refinement, and so as to aid in permanently gripping needle assembly 100 within housing 2 an engaging means may be provided so that lips 7 and 8 are retained permanently in the overlapping position. Such an engaging means is shown in Figure 6 as two opposing ratchets 6, 9, one on each lip 7, 8.

Whilst it is preferred that the apparatus 1 is a unitary moulded article (i.e. moulded in one piece), it is also possible to manufacture it from two or more different materials. For example, receiving portion 20 can be lined or otherwise provided with a grip enhancing material such as rubber, so as to increase the friction between hub 120 and receiving portion 20 (so as to aid in the prevention of rotation between hub 120 and receiving portion 20).

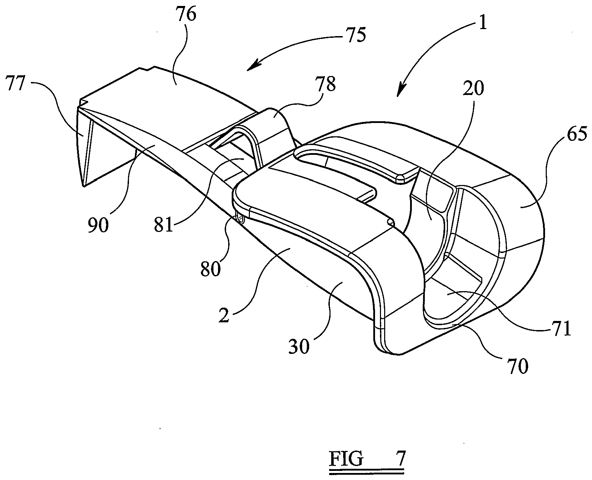

An embodiment is illustrated in Figures 7 to 10. The general principle of this embodiment is essentially the same as that of the needle removing apparatus described before, i.e. the apparatus 1 comprises a housing 2 with sides 30 and receiving portion 20 for receiving hub 120 of a needle assembly 100.

Figure 7 shows the apparatus of the embodiment in an open configuration. If it is moulded from plastics material it could for example leave the mould in that configuration.

Whilst in the first needle removing apparatus the bottom portion of the housing (i.e. opposite the slot 5, 10, 12) is closed, it is substantially open in the embodiment. In particular, the embodiment is formed with an opening 71 between the receiving portion 20 and a bridge portion 70 joining the sides 30 at the proximal end. However, an arm 75 is connected to the remainder of the housing at the distal end by means of e.g. a film hinge 80. The arm 75 comprises a relatively flat portion 76 which extends primarily in axial direction and, joined onto the distal end of portion 76 at approximately right angle thereto, a shorter portion 77. Portion 76 as shown in Figure 7 will be referred to as "horizontal portion" and portion 77 will be referred to as "vertical portion".

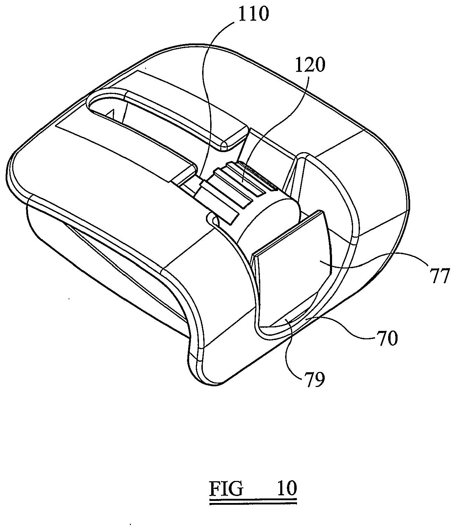

Arm 75 can be pivoted about hinge 80 by approximately 180° in counter-clockwise direction (as seen in Figure 7) so that it substantially covers the open bottom of housing 2, as illustrated in Figure 10. As shown in Figure 10, when arm 75 is pivoted towards the closed configuration shown in Figure 10, vertical portion 77 projects through opening 71 so as to block axial access to hub 120 from the proximal end.

Arm 75 is further formed with side portions 90 also extending at approximately 90° from horizontal portion 76, along the lateral edges of horizontal portion 76. This not only provides arm 75 with some degree of stability, but also complements the shape of sides 30 of housing 2 so that, in the closed configuration of Figure 10, sides 30 and side portions 90 form a substantially smooth surface which closes the apparatus laterally.

Arm 75 is further provided with an opening 81 at the centre of its proximal end (as seen in Figure 7). A tongue 78 extends between the proximal and distal end of opening 81.

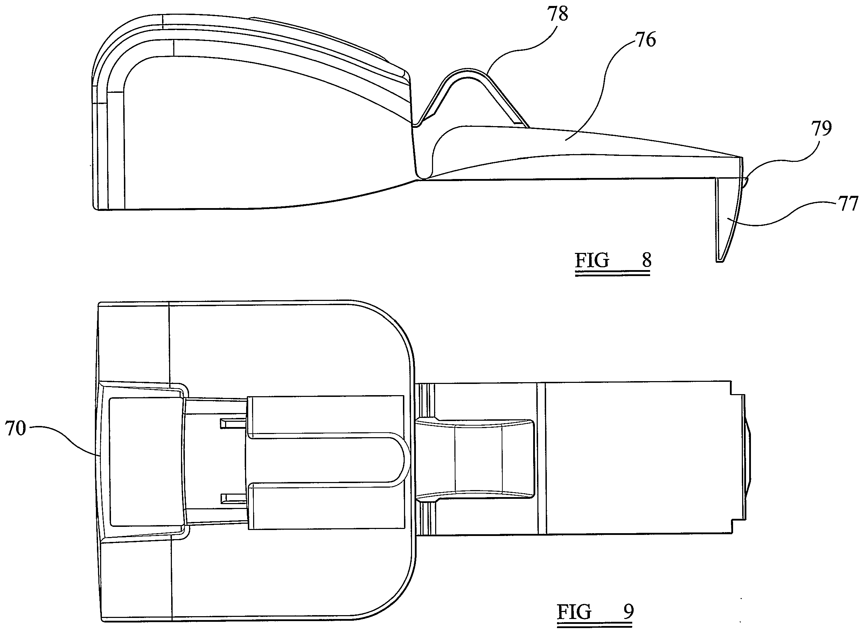

At its distal end, tongue 78 is joined onto horizontal portion 76, whereas its proximal end is joined onto the distal end of housing 2 (approximately in line with hinge 80). The connection between tongue 78 and horizontal portion 76 on the one hand and housing 2 on the other hand is also achieved by means of a film hinge. As best seen in Figure 8, tongue 78 has a generally "V" shape section. That is, the crest of this "V" shape projects upwards (as seen in Figure 7) when the apparatus is in the open configuration. Tongue 78 is "bistable", i.e. in the absence of external forces the apparatus 1 would remain in the open configuration shown in Figures 7 to 9, but, once brought into the closed configuration shown in Figure 10, the apparatus 1 would also remain in that configuration, in the absence of external forces.

Once the apparatus has been brought into the closed configuration the tongue 78 approximately covers the entire opening 81. Preferably, any gap between the sides of tongue 78 and those portions of horizontal portion 76 which are located adjacent the opening 81 is narrower than the diameter of a typical needle 110.

Finally, arm 75 is formed with a locking projection 79 (best seen in Figure 8), where the horizontal and vertical portions 76, 77 join. The generally wedge shaped locking projection 79 projects slightly from the vertical portion 77 in distal direction (as seen in Figure 8). As the apparatus 1 is transferred from the open configuration into the closed configuration the vertical portion 77 moves past bridge 70 with only a minimal gap therebetween. The pivoting movement of arm 75 is temporarily stopped when locking projection 79 "strikes" bridge 70 from underneath. The biasing force exerted by tongue 78 is not strong enough to force locking projection 79 past bridge 70, i.e. once most of the pivoting movement has been performed, the arm 75 would remain in an intermediate configuration (not shown in the drawings) where locking projection 79 buts against bridge 70 from underneath. Only once sufficient force is exerted onto the apparatus, e.g. by holding the apparatus 1 between a user's thumb and index and middle fingers (the thumb pressing upwards onto the horizontal portion 76 and the index and middle fingers pressing downwards onto the top of housing 2, adjacent the slot 5, 10, 12). When sufficient force is exerted the bridge 70 slides past locking projection 79, using the wedge shaped locking projection 79 as a ramp. Once this sliding movement has been completed the apparatus is in the closed configuration shown in Figure 10. The sliding movement is completed by locking projection 79 "clicking" into place once it has passed bridge 70, and bridge 70 cannot then easily slide back past locking projection 79 since locking projection 79 is formed with a ramp only on one side.

As a further difference to the first needle removing apparatus, the embodiment is formed with a finger protection 65 which, in the embodiment shown in the drawings, comprises a flange adjacent to the slot 5, 10, 12 and the proximal opening. When apparatus 1 is held between a user's thumb and index finger (the two sides 30 of housing 2 between thumb and index finger) the flange 65 covers a substantial portion of the user's fingers so as to protect the user against injury during the insertion process.

The insertion process of the embodiment can be carried out as follows. The apparatus 1 may be supplied in the intermediate configuration (not shown), i.e. the configuration where the locking projection 79 buts against bridge 70 from underneath. Vertical portion 77 partially obstructs the axial opening of housing 2 at its proximal end. The user would then hold housing 2 between thumb and index finger of one hand whilst holding an injecting device 200 with mounted needle assembly 100 in the other hand. The needle assembly 100 and the front portion of the injecting device 200 is then inserted through slot 5, 10, 12 into housing 2 as has been described in connection with the first needle removing apparatus. As hub 120 enters the receiving portion 20 the front portion 230 of injecting device 200 pushes down onto vertical portion 77 so as to pivot arm 75 away from housing 2. This can be done with relative ease since only the relatively weak biasing force of tongue 78 needs to be overcome. However, during insertion of needle assembly 100 and front portion 230 of injecting device 200 arm 75 remains biased towards the closed configuration, i.e. it is not "flipped" into the open configuration.

Once hub 120 has been received in receiving portion 20 the injecting device 200 is separated from needle assembly 100 by unthreading the threaded connection and axially withdrawing the injecting device 200 in proximal direction relative to needle assembly 100. If necessary, the grip between receiving portion 20 and hub 120 can be increased by the application of lateral pressure onto sides 30. As injecting device 200 is withdrawn axially it no longer pushes down onto vertical portion 77 so that the apparatus returns to the intermediate configuration where locking projection 79 buts against bridge 70 from underneath. The user can then bring apparatus 1 into the closed configuration by squeezing the apparatus 1 between thumb, index and middle finger, as described above. Once locking projection 79 has moved past bridge 70 axial access to hub 120 is substantially blocked. Hence it is virtually impossible to reconnect needle assembly 100 and injecting device 200. Needle assembly 100 is then held securely within closed housing 2 and can safely be disposed of, together with apparatus 1.

Although the invention has been described in terms of preferred embodiments as set forth above, it should be understood that these embodiments are illustrative only and that the claims are not limited to those embodiments. Those skilled in the art will be able to make modifications and alternatives in view of the disclosure which are contemplated as falling within the scope of the appended claims. Each feature disclosed or illustrated in the present specification may be incorporated in the invention, whether alone or in any appropriate combination with any other feature disclosed or illustrated herein.

## Claims

1. Apparatus for removing a needle assembly from an injecting device, the needle assembly comprising a needle holder portion and a needle portion secured to, or integral with, the needle holder portion, the needle assembly being attachable to the injecting device via the needle holder portion, the apparatus comprising
a housing (2) having an opening (5, 10, 12) through which the needle assembly can enter the interior of the housing, wherein within the housing there is provided a receiving portion (20) which is arranged to receive the needle holder portion in such a way as to impede rotation of the needle holder portion with respect to the receiving portion (20) about the axis of the needle, and wherein the opening (5, 10, 12) is formed such that the needle portion can enter the housing (2) and the needle holder portion can enter the receiving portion (20) by means of a relative movement between the housing (2) and the needle portion which relative movement is lateral with respect to the axis of the needle portion, **characterised in that** the housing (2) is resiliently deformable,
the apparatus further comprising an arm (75) pivotally connected to the housing at the distal end of the housing;
the arm (75) comprising a first portion (76) and a second portion (77), whereby the second portion is joined to the first portion at substantially a right angle;
an opening (71) between the receiving portion (20) and a bridge portion (70), wherein the bridge portion joins sides (30) at a proximal end;
wherein the second portion (77), when pivoted towards a closed configuration, projects through the opening (71) and blocks access to the needle holder portion (120) from the proximal end of the needle holder portion,
wherein the arm (75) comprises an opening (81) at the centre of its proximal end and a tongue (78) which extends between the proximal end and the distal end of the opening (81);
wherein one end of the tongue is joined onto the first portion (76) and wherein the other end of the tongue in joined to the distal end of the housing (2);
wherein the tongue has substantially a V shape such that the tongue is bistable and such that the apparatus remains in an open configuration in the absence of external forces if the apparatus is in the open configuration and
remains in a closed configuration in the absence of external forces if the apparatus is in the closed configuration.

2. Apparatus according to claim 1, wherein the opening (5, 10, 12) comprises a slot (5, 10, 20) at one side of the housing.

3. Apparatus according to claim 2, wherein the slot is of non-uniform width.

4. Apparatus according to claim 3, wherein the slot comprises a first slot portion (5) through which the needle portion is arranged to pass, and a second slot portion (10, 12) through which the needle holder portion is arranged to pass, wherein the second slot portion is wider, preferably substantially wider than the first slot portion.

5. Apparatus according to claim 4, wherein the second slot portion (10, 12) is at least twice as wide as the first slot portion (5).

6. Apparatus according to claim 5, wherein the second slot portion (10, 12) is at least 8mm wide, preferably at least 9mm wide.

7. Apparatus according to claim 4, wherein the first slot portion (5) is less than 5mm wide, preferably less than 4mm wide.

8. Apparatus according to any of claims 1 to 7, wherein the apparatus is resiliently deformable.

9. Apparatus according to claim 8 as dependent on any of claims 2 to 7, wherein, in the relaxed state of the apparatus, the slot (5, 10, 12) is open sufficiently wide so as to permit the needle portion and the needle holder portion to pass through the slot.

10. Apparatus according to claim 8, wherein the apparatus is arranged to deform resiliently as the needle holder portion enters the receiving portion.

11. Apparatus according to any of claims 1 to 10, wherein the housing (2) is formed so as to be able to surround the entire needle holder portion.

12. Apparatus according to any of claims 1 to 11, wherein the housing (2) is formed so as to be able to surround the entire needle assembly.

13. Apparatus according to any of claims 1 to 12, wherein the needle holder portion is formed with an internal thread for connection to an external thread formed on the injecting device.

14. Apparatus according to claim 13, wherein the thread of the injecting device is a standard thread for insulin injecting devices.

15. Apparatus according to claim 13, wherein the thread of the injecting device is a thread of the type suitable for use with insulin injecting devices complying with ISO 11608.

16. Apparatus according to claim 13, wherein the thread of the injecting device has an external diameter of 9.5mm and has a pitch of 32 threads per inch (32 threads per 2.54cm).

17. Apparatus according to any of claims 1 to 16, wherein the external diameter of the needle holder portion is between 10 and 14mm, preferably between 10 and 13mm, more preferably between 10 and 12mm and most preferably between 11 and 12 mm.

18. Apparatus according to any of claims 1 to 17, wherein the receiving portion (20) is formed with at least one longitudinal rib for inhibiting said rotation.

19. Apparatus according to any of claims 1 to 18, wherein the receiving portion (20) is formed with a friction-increasing material for inhibiting said rotation.

20. Apparatus according to claim 19, wherein at least one portion of the apparatus other than said receiving portion (20) is formed with a material which has a lower coefficient of friction than said friction-increasing material.

21. Apparatus according to any of claims 1 to 19, wherein the apparatus is a unitary moulded article.

22. Apparatus according to any of claims 1 to 21, comprising first impeding means for impeding removal of the needle assembly from the apparatus.

23. Apparatus according to claim 22, wherein the first impeding means comprises a neck portion between the opening and the receiving portion which has a smaller passage width than the receiving portion.

24. Apparatus according to any of claims 1 to 23, further comprising second impeding means (40, 45) for impeding re-attachment of the injecting device to the needle holder portion once the needle assembly has been removed from the injecting device.

25. Apparatus according to claim 24, wherein the second impeding means (40, 45) comprises at least one radially inwardly projecting portion located towards one axial end of the apparatus.

26. Apparatus according to claim 25, wherein the inwardly projecting portion is displaceable outwardly so that it is biased inwardly.

27. Apparatus according to claim 26, wherein the second impeding means comprises at least two such radially inwardly projecting portions (40, 45) generally opposing each other.

28. Apparatus according to claim 27, wherein the largest circle which can be placed between said inwardly projecting portions in their relaxed state has a diameter which is smaller than the diameter of that portion of the injecting device which is immediately adjacent to that portion of the injecting device to which the needle holder portion is arranged to be attached.

29. Apparatus according to claim 24, wherein the second impeding means comprises means for substantially blocking access to the proximal end of the needle holder assembly in axial direction when the needle holder portion has been received in the receiving portion.

30. Apparatus according to claim 29, wherein the blocking means comprising a member which is connected to the remainder of the apparatus by means of a hinge so that it is movable between a first position in which it does not block said access and a second position in which it does block said access.

31. Apparatus according to claim 30, wherein the blocking means comprises locking means for locking the member in the second position.

32. Apparatus according to claim 2, or any of claims 3 to 31 as directly or indirectly dependent on claim 2, wherein at least a portion of the slot is formed by two generally opposing lips located at different radial positions of the apparatus.

33. Apparatus according to claim 32, wherein the apparatus can be squeezed such that the two lips overlap.

34. Apparatus according to claim 33, further comprising engaging means for engaging the lips with each other when they have been caused to overlap.

35. Apparatus according to any of claims 1 to 34, further comprising a shield (65) for protecting the fingers of a user when holding the apparatus.

36. Apparatus according to claim 35 wherein the shield (65) comprises a flange adjacent the opening.

37. A system comprising the apparatus of any of claims 1 to 36 and a said needle assembly.

38. A system according to claim 37, further comprising a said injecting device to which said needle assembly is attached.

39. A method of sheathing a needle assembly attached to an injecting device, comprising:
providing a system according to claim 38;
performing a relative movement between the housing (2) and the needle portion which relative movement is lateral with respect to the axis of the needle portion so that the needle assembly enters the interior of the housing (2), and so that the needle holder portion enters the receiving portion by means of said relative movement.

40. A method of removing a needle assembly from an injecting device, comprising:
performing the method according to claim 39;
rotating the apparatus with respect to the injecting device; and
removing the apparatus axially from the injecting device so as to axially remove the needle assembly from the injecting device.

41. A method according to claim 39 or 40, wherein, when the needle holder portion is attached to the injecting device, there is substantially no gap between the injecting device and that surface of the needle holder portion which is furthest away from the tip of the needle portion.

## Patentansprüche

1. Vorrichtung zum Entfernen einer Nadelbaugruppe von einer Injektionsvorrichtung, wobei die Nadelbaugruppe einen Nadelhalterabschnitt und einen Nadelabschnitt, der an dem Nadelhalterabschnitt befestigt oder integral mit demselben ist, umfasst, wobei die Nadelbaugruppe über den Nadelhalterabschnitt an der Injektionsvorrichtung befestigt werden kann, wobei die Vorrichtung Folgendes umfasst:
ein Gehäuse (2), das eine Öffnung (5, 10, 12) hat, durch welche die Nadelbaugruppe in das Innere des Gehäuses eintreten kann, wobei innerhalb des Gehäuses ein Aufnahmeabschnitt (20) bereitgestellt wird, der dafür angeordnet ist, den Nadelhalterabschnitt auf eine solche Weise aufzunehmen, dass eine Drehung des Nadelhalterabschnitts in Bezug auf den Aufnahmeabschnitt (20) um die Achse der Nadel verhindert wird, und wobei die Öffnung (5, 10, 12) derart geformt ist, dass der Nadelabschnitt in das Gehäuse (2) eintreten kann und der Nadelhalterabschnitt in den Aufnahmeabschnitt (20) eintreten kann, mit Hilfe einer relativen Bewegung zwischen dem Gehäuse (2) und dem Nadelabschnitt, wobei die relative Bewegung seitlich in Bezug auf die Achse des Nadelabschnitts ist, **dadurch gekennzeichnet, dass** das Gehäuse (2) elastisch verformbar ist,
wobei die Vorrichtung ferner einen Arm (75) umfasst, der an dem distalen Ende des Gehäuses schwenkbar mit dem Gehäuse verbunden ist,
wobei der Arm (75) einen ersten Abschnitt (76) und einen zweiten Abschnitt (77) umfasst, wobei der zweite Abschnitt in einem im Wesentlichen rechten Winkel mit dem ersten Abschnitt verbunden ist,
eine Öffnung (71) zwischen dem Aufnahmeabschnitt (20) und einem Brückenabschnitt (70), wobei der Brückenabschnitt Seiten (30) an einem proximalen Ende verbindet,
wobei der zweite Abschnitt (77), wenn er zu einer geschlossenen Konfiguration hin geschwenkt wird, durch die Öffnung (71) vorspringt und einen Zugang zu dem Nadelhalterabschnitt (120) von dem proximalen Ende des Nadelhalterabschnitts sperrt,
wobei der Arm (75) eine Öffnung (81) an der Mitte seines proximalen Endes und eine Zunge (78), die sich zwischen dem proximalen Ende und dem distalen Ende der Öffnung (81) erstreckt, umfasst,
wobei das eine Ende der Zunge mit dem ersten Abschnitt (76) verbunden ist und wobei das andere Ende der Zunge mit dem distalen Ende des Gehäuses (2) verbunden ist,
wobei die Zunge im Wesentlichen eine V-Form hat, derart, dass die Zunge bistabil ist, und derart, dass die Vorrichtung beim Fehlen von äußeren Kräften in einer offenen Konfiguration bleibt, falls sich die Vorrichtung in der offenen Konfiguration befindet, und beim Fehlen von äußeren Kräften in einer geschlossenen Konfiguration bleibt, falls sich die Vorrichtung in der geschlossenen Konfiguration befindet.

2. Vorrichtung nach Anspruch 1, wobei die Öffnung (5, 10, 12) einen Schlitz (5, 10, 12) an einer Seite des Gehäuses umfasst.

3. Vorrichtung nach Anspruch 2, wobei der Schlitz eine ungleichförmige Breite hat.

4. Vorrichtung nach Anspruch 3, wobei der Schlitz einen ersten Schlitzabschnitt (5), durch den hindurchzugehen der Nadelabschnitt angeordnet ist, und einen zweiten Schlitzabschnitt (10, 12), durch den hindurchzugehen der Nadelhalterabschnitt angeordnet ist, umfasst, wobei der zweite Schlitzabschnitt breiter, vorzugsweise wesentlich breiter, ist als der erste Schlitzabschnitt.

5. Vorrichtung nach Anspruch 4, wobei der zweite Schlitzabschnitt (10, 12) wenigstens zweimal so breit ist wie der erste Schlitzabschnitt (5).

6. Vorrichtung nach Anspruch 5, wobei der zweite Schlitzabschnitt (10, 12) wenigstens 8 mm breit ist, vorzugsweise wenigstens 9 mm breit ist.

7. Vorrichtung nach Anspruch 4, wobei der erste Schlitzabschnitt (5) weniger als 5 mm, vorzugsweise weniger als 4 mm breit ist.

8. Vorrichtung nach einem der Ansprüche 1 bis 7, wobei die Vorrichtung elastisch verformbar ist.

9. Vorrichtung nach Anspruch 8, soweit abhängig von einem der Ansprüche 2 bis 7, wobei, in dem entspannten Zustand der Vorrichtung, der Schlitz (5, 10, 12) ausreichend weit offen ist, um so zu ermöglichen, dass der Nadelabschnitt und der Nadelhalterabschnitt durch den Schlitz hindurchgehen.

10. Vorrichtung nach Anspruch 8, wobei die Vorrichtung dafür angeordnet ist, sich elastisch zu verformen, wenn der Nadelhalterabschnitt in den Aufnahmeabschnitt eintritt.

11. Vorrichtung nach einem der Ansprüche 1 bis 10, wobei das Gehäuse (2) so geformt ist, dass es dazu in der Lage ist, den gesamten Nadelhalterabschnitt zu umgeben.

12. Vorrichtung nach einem der Ansprüche 1 bis 11, wobei das Gehäuse (2) so geformt ist, dass es dazu in der Lage ist, die gesamte Nadelhalterbaugruppe zu umgeben.

13. Vorrichtung nach einem der Ansprüche 1 bis 12, wobei der Nadelhalterabschnitt mit einem Innengewinde geformt ist, zur Verbindung mit einem Außengewinde, das an der Injektionsvorrichtung geformt ist.

14. Vorrichtung nach Anspruch 13, wobei das Gewinde der Injektionsvorrichtung ein standardmäßiges Gewinde für Insulin-Injektionsvorrichtungen ist.

15. Vorrichtung nach Anspruch 13, wobei das Gewinde der Injektionsvorrichtung ein Gewinde der Art ist, die zur Verwendung mit Insulin-Injektionsvorrichtungen geeignet ist, die ISO 11608 entsprechen.

16. Vorrichtung nach Anspruch 13, wobei das Gewinde der Injektionsvorrichtung einen Außendurchmesser von 9,5 mm hat und eine Teilung von 32 Gewindegängen pro Zoll (32 Gewindegängen pro 2,54 cm) hat.

17. Vorrichtung nach einem der Ansprüche 1 bis 16, wobei der Außendurchmesser des Nadelhalterabschnitts zwischen 10 und 14 mm, vorzugsweise zwischen 10 und 13 mm, insbesondere zwischen 10 und 12 mm und am bevorzugtesten zwischen 11 und 12 mm beträgt.

18. Vorrichtung nach einem der Ansprüche 1 bis 17, wobei der Aufnahmeabschnitt (20) mit wenigstens einer Längsrippe zum Hemmen der Drehung geformt ist.

19. Vorrichtung nach einem der Ansprüche 1 bis 18, wobei der Aufnahmeabschnitt (20) mit einem reibungssteigernden Material zum Hemmen der Drehung geformt ist.

20. Vorrichtung nach Anspruch 19, wobei wenigstens ein anderer Abschnitt der Vorrichtung als der Aufnahmeabschnitt (20) mit einem Material geformt ist, das einen niedrigeren Reibungskoeffizienten hat als das reibungssteigernde Material.

21. Vorrichtung nach einem der Ansprüche 1 bis 19, wobei die Vorrichtung ein einteiliger geformter Artikel ist.

22. Vorrichtung nach einem der Ansprüche 1 bis 21, das erste Verhinderungsmittel zum Verhindern eines Entfernens der Nadelbaugruppe von der Vorrichtung umfasst.

23. Vorrichtung nach Anspruch 22, wobei das erste Verhinderungsmittel einen Halsabschnitt zwischen der Öffnung und dem Aufnahmeabschnitt umfasst, der eine kleinere Durchgangsbreite hat als der Aufnahmeabschnitt.

24. Vorrichtung nach einem der Ansprüche 1 bis 23, das ferner ein zweites Verhinderungsmittel (40, 45) zum Verhindern einer Wiederbefestigung der Injektionsvorrichtung an dem Nadelhalterabschnitt, sobald die Nadelbaugruppe von der Injektionsvorrichtung entfernt worden ist, umfasst.

25. Vorrichtung nach Anspruch 24, wobei das zweite Verhinderungsmittel (40, 45) wenigstens einen in Radialrichtung nach innen vorspringenden Abschnitt umfasst, der zu einem axialen Ende der Vorrichtung hin angeordnet ist.

26. Vorrichtung nach Anspruch 25, wobei der nach innen vorspringende Abschnitt nach außen verschiebbar ist, so dass er nach innen vorgespannt wird.

27. Vorrichtung nach Anspruch 26, wobei das zweite Verhinderungsmittel wenigstens zwei solcher in Radialrichtung nach innen vorspringenden Abschnitte (40, 45) umfasst, die einander im Allgemeinen gegenüberliegen.

28. Vorrichtung nach Anspruch 27, wobei der größte Kreis, der zwischen den nach innen vorspringenden Abschnitten in ihrem entspannten Zustand platziert werden kann, einen Durchmesser hat, der kleiner ist als der Durchmesser des Abschnitts der Injektionsvorrichtung, der unmittelbar dem Abschnitt der Injektionsvorrichtung benachbart ist, an dem befestigt zu werden der Nadelhalterabschnitt angeordnet ist.

29. Vorrichtung nach Anspruch 24, wobei das zweite Verhinderungsmittel ein Mittel umfasst, um einen Zugang zu dem proximalen Ende der Nadelhalterbaugruppe in axialer Richtung im Wesentlichen zu sperren, wenn der Nadelhalterabschnitt in dem Aufnahmeabschnitt aufgenommen worden ist.

30. Vorrichtung nach Anspruch 29, wobei das Sperrmittel ein Element umfasst, das mit dem Rest der Vorrichtung mit Hilfe eines Scharniers verbunden ist, so dass es beweglich ist zwischen einer ersten Stellung, in der es den Zugang nicht sperrt, und einer zweiten Stellung, in der es den Zugang sperrt.

31. Vorrichtung nach Anspruch 30, wobei das Sperrmittel ein Arretierungsmittel zum Arretieren des Elements in der zweiten Stellung umfasst.

32. Vorrichtung nach Anspruch 2 oder einem der Ansprüche 3 bis 31, soweit unmittelbar oder mittelbar abhängig von Anspruch 2, wobei wenigstens ein Abschnitt des Schlitzes durch zwei im Allgemeinen gegenüberliegende Lippen gebildet wird, die an unterschiedlichen radialen Positionen der Vorrichtung angeordnet sind.

33. Vorrichtung nach Anspruch 32, wobei die Vorrichtung derart gequetscht werden kann, dass die zwei Lippen überlappen.

34. Vorrichtung nach Anspruch 33, die ferner ein Eingriffsmittel zum In-Eingriff-Bringen der Lippen miteinander, wenn sie veranlasst worden sind, zu überlappen, umfasst.

35. Vorrichtung nach einem der Ansprüche 1 bis 34, das ferner einen Schutz (65) zum Schützen der Finger eines Benutzers, wenn er die Vorrichtung hält, umfasst.

36. Vorrichtung nach Anspruch 35, wobei der Schutz (65) einen Flansch angrenzend an die Öffnung umfasst.

37. System, das die Vorrichtung nach einem der Ansprüche 1 bis 36 und eine Nadelbaugruppe umfasst.

38. System nach Anspruch 37, das ferner eine Injektionsvorrichtung umfasst, an der die Nadelbaugruppe befestigt ist.

39. Verfahren zum Ummanteln einer Nadelbaugruppe, die an einer Injektionsvorrichtung befestigt ist, das Folgendes umfasst:
das Bereitstellen eines Systems nach Anspruch 38,
das Durchführen einer relativen Bewegung zwischen dem Gehäuse (2) und dem Nadelabschnitt, wobei die relative Bewegung seitlich in Bezug auf die Achse des Nadelabschnitts ist, so dass die Nadelbaugruppe in das Gehäuse (2) eintritt und so dass der Nadelhalterabschnitt mit Hilfe der relativen Bewegung in den Aufnahmeabschnitt eintritt.

40. Verfahren zum Entfernen einer Nadelbaugruppe von einer Injektionsvorrichtung, das Folgendes umfasst:
das Ausführen des Verfahrens nach Anspruch 39,
das Drehen der Vorrichtung in Bezug auf die Injektionsvorrichtung und
das Entfernen der Vorrichtung von der Injektionsvorrichtung in Axialrichtung, um so die Nadelbaugruppe in Axialrichtung von der Injektionsvorrichtung zu entfernen.

41. Verfahren nach Anspruch 39 oder 40, wobei, wenn der Nadelhalterabschnitt an der Injektionsvorrichtung befestigt ist, es im Wesentlichen keinen Spalt zwischen der Injektionsvorrichtung und der Oberfläche des Nadelhalterabschnitts, die am weitesten von der Spitze des Nadelabschnitts entfernt ist, gibt.

## Revendications

1. Dispositif permettant de retirer un ensemble à aiguille d'un instrument d'injection, l'ensemble à aiguille comprenant une partie porte-aiguille et une partie formant aiguille fixée à la partie porte-aiguille, ou constituée d'un seul tenant avec cette dernière, l'ensemble à aiguille pouvant être fixé sur l'instrument d'injection par la partie porte-aiguille, le dispositif comprenant :
une enveloppe (2) présentant une ouverture (5, 10, 12) à travers laquelle l'ensemble à aiguille peut pénétrer à l'intérieur de l'enveloppe, une partie réceptrice (20) étant prévue dans l'enveloppe en étant agencée pour recevoir la partie porte-aiguille de manière à empêcher la rotation de la partie porte-aiguille par rapport à la partie réceptrice (20) autour de l'axe de l'aiguille, et l'ouverture (5, 10, 12) étant constituée de manière que la partie formant aiguille puisse pénétrer dans l'enveloppe (2) et la partie porte-aiguille puisse pénétrer dans la partie réceptrice (20) sous l'effet d'un mouvement relatif entre l'enveloppe (2) et la partie formant aiguille, lequel mouvement relatif est latéral par rapport à l'axe de la partie formant aiguille ; **caractérisé en ce que** l'enveloppe (2) est élastiquement déformable ;
le dispositif comprenant en outre un bras (75) relié pivotant à l'enveloppe, à l'extrémité distale de celle-ci ;
le bras (75) comprenant une première partie (76) et une deuxième partie (77), ladite deuxième partie étant jointe à la première partie en formant sensiblement un angle droit ;
une ouverture (71) entre la partie réceptrice (20) et une partie de liaison (70), ladite partie de liaison reliant les côtés (30) sur une extrémité proximale ;
dans lequel la deuxième partie (77), ayant subi un pivotement pour prendre une configuration fermée, fait saillie par l'ouverture (71) et bloque l'accès à la partie porte-aiguille (120) par l'extrémité proximale de la partie porte-aiguille,
dans lequel le bras (75) comprend une ouverture (81) au centre de son extrémité proximale et une languette (78) comprise entre l'extrémité proximale et l'extrémité distale de l'ouverture (81) ;
dans lequel une extrémité de la languette est jointe à la première partie (76) et l'autre extrémité de la languette est jointe à l'extrémité distale de l'enveloppe (2) ;
dans lequel la languette présente une forme sensiblement en V si bien qu'elle est bistable et qu'elle permet au dispositif de rester dans une configuration ouverte en l'absence de forces extérieures si le dispositif se trouve dans la configuration ouverte et de rester dans une configuration fermée en l'absence de forces extérieures si le dispositif se trouve dans la configuration fermée.

2. Dispositif selon la revendication 1, dans lequel l'ouverture (5, 10, 12) comprend une fente (5, 10, 12) sur un côté de l'enveloppe.

3. Dispositif selon la revendication 2, dans lequel la fente est de largeur non uniforme.

4. Dispositif selon la revendication 3, dans lequel la fente comprend une première partie de fente (5) à travers laquelle la partie formant aiguille est conçue pour passer, et une deuxième partie de fente (10, 12) à travers laquelle la partie porte-aiguille est conçue pour passer, ladite deuxième partie de fente étant plus large, de préférence sensiblement plus large, que la première partie de fente.

5. Dispositif selon la revendication 4, dans lequel la deuxième partie de fente (10, 12) est au moins deux fois plus large que la première partie de fente (5).

6. Dispositif selon la revendication 5, dans lequel la deuxième partie de fente (10, 12) fait au moins 8 mm de large, de préférence au moins 9 mm de large.

7. Dispositif selon la revendication 4, dans lequel la première partie de fente (5) fait moins de 5 mm de large, de préférence moins de 4 mm de large.

8. Dispositif selon l'une quelconque des revendications 1 à 7, dans lequel le dispositif est élastiquement déformable.

9. Dispositif selon la revendication 8, dans la mesure où elle dépend de l'une quelconque des revendications 2 à 7, dans lequel, le dispositif étant à l'état détendu, la fente (5, 10, 12) est ouverte suffisamment large pour permettre à la partie formant aiguille et à la partie porte-aiguille de traverser la fente.

10. Dispositif selon la revendication 8, dans lequel le dispositif est conçu pour se déformer de manière élastique tandis que la partie porte-aiguille pénètre dans la partie réceptrice.

11. Dispositif selon l'une quelconque des revendications 1 à 10, dans lequel l'enveloppe (2) est constituée de manière à entourer la totalité de la partie porte-aiguille.

12. Dispositif selon l'une quelconque des revendications 1 à 11, dans lequel l'enveloppe (2) est constituée de manière à entourer la totalité de l'ensemble à aiguille.

13. Dispositif selon l'une quelconque des revendications 1 à 12, dans lequel la partie porte-aiguille est pourvue d'un filet intérieur destiné à coopérer avec un filet extérieur créé sur l'instrument d'injection.

14. Dispositif selon la revendication 13, dans lequel le filet de l'instrument d'injection est un filet normalisé pour les instruments d'injection d'insuline.

15. Dispositif selon la revendication 13, dans lequel le filet de l'instrument d'injection est un filet de type approprié aux instruments d'injection d'insuline conformes à la norme ISO 11608.

16. Dispositif selon la revendication 13, dans lequel le filet de l'instrument d'injection présente un diamètre extérieur de 9,5 mm et un pas de 32 filets par pouce (32 filets pour 2,54 cm).

17. Dispositif selon l'une quelconque des revendications 1 à 16, dans lequel le diamètre extérieur de la partie porte-aiguille est compris entre 10 et 14 mm, de préférence entre 10 et 13 mm, plus préférablement entre 10 et 12 mm et tout préférablement entre 11 et 12 mm.

18. Dispositif selon l'une quelconque des revendications 1 à 17, dans lequel la partie réceptrice (20) est pourvue d'au moins une nervure longitudinale visant à entraver ladite rotation.

19. Dispositif selon l'une quelconque des revendications 1 à 18, dans lequel la partie réceptrice (20) présente un matériau à effet d'augmentation du frottement visant à entraver ladite rotation.

20. Dispositif selon la revendication 19, dans lequel au moins une partie du dispositif autre que ladite partie réceptrice (20) présente un matériau offrant un moindre coefficient de frottement que ledit matériau à effet d'augmentation du frottement.

21. Dispositif selon l'une quelconque des revendications 1 à 19, dans lequel le dispositif est un article moulé d'une seule pièce.

22. Dispositif selon l'une quelconque des revendications 1 à 21, comprenant un premier moyen d'empêchement visant à empêcher le retrait de l'ensemble à aiguille du dispositif.

23. Dispositif selon la revendication 22, dans lequel le premier moyen d'empêchement comprend une partie de collet, entre l'ouverture et la partie réceptrice, qui présente une plus petite largeur de passage que la partie réceptrice.

24. Dispositif selon l'une quelconque des revendications 1 à 23, comprenant en outre un second moyen d'empêchement (40, 45) visant à empêcher que l'instrument d'injection se fixe de nouveau à la partie porte-aiguille une fois que l'ensemble à aiguille a été retiré de l'instrument d'injection.

25. Dispositif selon la revendication 24, dans lequel le deuxième moyen d'empêchement (40, 45) comprend au moins une partie en saillie radiale vers l'intérieur située vers une extrémité axiale du dispositif.

26. Dispositif selon la revendication 25, dans lequel la partie en saillie vers l'intérieur est déplaçable vers l'extérieur si bien qu'elle est sollicitée vers l'intérieur.

27. Dispositif selon la revendication 26, dans lequel le deuxième moyen d'empêchement comprend au moins deux de ces parties en saillie radiale vers l'intérieur (40, 45) globalement en vis-à-vis l'une de l'autre.

28. Dispositif selon la revendication 27, dans lequel le plus grand cercle pouvant être disposé entre lesdites parties en saillie vers l'intérieur dans leur état détendu présente un diamètre qui est inférieur au diamètre de la partie de l'instrument d'injection qui est immédiatement adjacente à la partie de l'instrument d'injection à laquelle la partie porte-aiguille est conçue pour être fixée.

29. Dispositif selon la revendication 24, dans lequel le deuxième moyen d'empêchement comprend un moyen visant à bloquer sensiblement l'accès à l'extrémité proximale de l'ensemble porte-aiguille dans le sens axial une fois la partie porte-aiguille reçue dans la partie réceptrice.

30. Dispositif selon la revendication 29, dans lequel le moyen de blocage comprend un élément relié au reste du dispositif au moyen d'une charnière si bien qu'il est mobile entre une première position dans laquelle il ne bloque pas ledit accès et une deuxième position dans laquelle il bloque ledit accès.

31. Dispositif selon la revendication 30, dans lequel le moyen de blocage comprend un moyen de verrouillage assurant le verrouillage de l'élément dans la deuxième position.

32. Dispositif selon la revendication 2, ou l'une quelconque des revendications 3 à 31 dans la mesure où elles dépendent directement ou indirectement de la revendication 2, dans lequel au moins une partie de la fente se compose de deux lèvres globalement opposées, situées en des positions radiales différentes du dispositif.

33. Dispositif selon la revendication 32, le dispositif pouvant être comprimé de manière que les deux lèvres de chevauchent.

34. Dispositif selon la revendication 33, comprenant en outre un moyen d'emboîtement assurant l'emboîtement des lèvres l'une avec l'autre une fois qu'elles ont été amenées en chevauchement.

35. Dispositif selon l'une quelconque des revendications 1 à 34, comprenant en outre une protection (65) visant à protéger les doigts d'un utilisateur tenant le dispositif.

36. Dispositif selon la revendication 35, dans lequel la protection (65) comprend un rebord adjacent à l'ouverture.

37. Système comprenant le dispositif selon l'une quelconque des revendications 1 à 36 et un dit ensemble à aiguille.

38. Système selon la revendication 37, comprenant en outre un dit instrument d'injection auquel est fixé ledit ensemble à aiguille.

39. Procédé visant à gainer un ensemble à aiguille fixé à un instrument d'injection, comprenant :
la mise à disposition d'un système selon la revendication 38 ;
la réalisation d'un mouvement relatif entre l'enveloppe (2) et la partie formant aiguille, lequel mouvement relatif est latéral par rapport à l'axe de la partie formant aiguille si bien que l'ensemble à aiguille pénètre à l'intérieur de l'enveloppe (2), et que la partie porte-aiguille pénètre dans la partie réceptrice sous l'effet dudit mouvement relatif.

40. Procédé de retrait d'un ensemble à aiguille d'un instrument d'injection, comprenant :
la réalisation du procédé selon la revendication 39 ;
la rotation du dispositif par rapport à l'instrument d'injection ; et
le retrait du dispositif, axialement, vis-à-vis de l'instrument d'injection afin de retirer axialement l'ensemble à aiguille de l'instrument d'injection.

41. Procédé selon les revendications 39 ou 40, dans lequel, lorsque la partie porte-aiguille est fixée à l'instrument d'injection, il ne se trouve sensiblement aucun espace entre l'instrument d'injection et la surface de la partie porte-aiguille qui est la plus éloignée de la pointe de la partie formant aiguille.
